# EUROPEAN PATENT APPLICATION

(11) **EP 4 678 602 A1**
(43) Date of publication of application: **14.01.2026**
(21) Application number: 25180714.5
(22) Date of filing: 04.06.2025
(51) Int. Cl.: C02F 1/32, C02F 1/467, C02F 1/461, C02F 9/20, C02F 103/02

(54) **PORTABLE DEVICE FOR REDOX THERAPY**

(30) Priority: 10.06.2024 ES 202431099 U
(71) Applicant: Camarero Villaverde, David, 64700 Biriatou (FR)
(72) Inventor: Camarero Villaverde, David, 64700 Biriatou (FR)
(74) Representative: Sánchez Paulino, Francisco Javier

(57) **Abstract**

The object of the invention is a portable device for redox therapy, which employs the technique of electrolysis in demineralised water with sodium chloride, along with ultraviolet light, in order to produce redox molecules with useful applications across various fields. It comprises a container (1) for the introduction of water with the amount of sodium chloride or saline solution, which contains electrodes (3) to carry out redox therapy by means of UV light radiation means (4).

## Description

### OBJECT OF THE INVENTION

The present invention, as the title establishes, is a portable device for redox therapy, which employs the technique of electrolysis in demineralised water with sodium chloride, together with ultraviolet light, in order to produce redox molecules with useful applications in different fields. This is an innovation that, within the current techniques, provides advantages that were unknown until now.

The present invention is characterised by the special functional and constructive features of the elements that make up the portable device for redox therapy, which enables the generation of redox molecules within a closed system, without the need for water input or output, through a process of electrolysis. It includes ultraviolet light-emitting means that accelerate the reactions, thereby allowing for a more efficient treatment.

### TECHNICAL SECTOR

Therefore, the present invention falls within the field of health.

### BACKGROUND OF THE INVENTION

Redox molecules are those that participate in oxidation-reduction reactions, a type of chemical reaction in which one or more electrons are transferred between chemical species. In these reactions, a chemical species is oxidised, that is, it loses electrons, while another is reduced, that is, it gains electrons. Redox molecules play a crucial role in numerous biological and chemical processes, including cellular respiration, photosynthesis, metal corrosion, and energy generation in cells and batteries. These molecules can be both organic and inorganic, and their ability to transfer electrons makes them fundamental to the functioning of biological systems and the synthesis of chemical compounds.

The production of redox molecules presents a series of challenges and issues that must be addressed carefully and precisely. Redox molecules, which are those capable of transferring electrons in chemical reactions, are fundamental in numerous biological, industrial, and environmental processes. However, its production entails significant challenges.

One of the main challenges in the production of redox molecules is the need to efficiently control the oxidation and reduction reactions. Given that these reactions involve the transfer of electrons, it is crucial to ensure they are carried out in a controlled manner to avoid the formation of undesired by-products or a reduction in efficiency. This requires the development of methods and processes that allow precise control of the reaction conditions, as well as the selection of catalysts and optimal operating conditions.

Another important challenge is the selection of suitable raw materials and reagents for the production of redox molecules. Given that these molecules are often used in critical applications, it is essential to ensure the purity and quality of the reagents used in their synthesis. This may involve the need to develop specific synthesis routes or to optimise existing processes to ensure the desired purity and efficiency.

Various patents have been developed for the production of redox molecules through electrolysis, including patents US5507932, US5674537, US6007686, and US6117285. These molecules include hypochlorous acid, hypochlorites, dissolved oxygen, chlorine, hydrogen gases, hydrogen peroxide, hydrogen, hypochlorite, superoxides, ozone, activated hydrogen, chloride ions, singlet oxygen hydroxides, and other reactive oxygen species. These techniques detail the configuration of the electrodes, and the continuous electric currents induced in them, as well as the forced mechanical movement of the brine solution through the electrodes.

In the device of the invention, a brine solution of water with sodium chloride, or even saline solution, is also used. A container with two electrodes is used, but no movement is forced upon the water, and a type C ultraviolet (UV) light is added to ensure its action on the oxygen is established between the two electrodes.

Regarding the present state of the art, it should be noted that, although various devices for generating redox molecules are known, none possess identical or similar structural and constitutive technical characteristics to those featured in the invention described herein.

### EXPLANATION OF THE INVENTION

The invention relates to a portable device for redox therapy, which uses the technique of electrolysis in demineralised water with sodium chloride or standardised saline solution, combined with UVC light, to obtain redox molecules useful in various fields. Unlike previously developed models, this new approach aims to achieve specific concentrations of redox molecules without the need for mechanical movement of water with sodium chloride.

The process begins with the use of a sealed container in which no movement is applied onto the water, but rather it is generated by the bubbling produced during electrolysis. Starting from a commercial saline solution or demineralised water with sodium chloride, different concentrations of redox molecules can be obtained by adding varying amounts of bidistilled and demineralised water with different concentrations of sodium chloride. This allows for obtaining more or less diluted concentrations of redox molecules according to therapeutic requirements.

The combination of electrolysis and UVC light in this portable device offers an effective method for the controlled production of redox molecules, which can have applications in the therapeutic, cosmetic, and food sectors, among others. This innovative approach offers significant advantages in terms of accuracy in achieving desired concentrations, as well as in the simplicity and portability of the device.

Moreover, the ability to adjust the concentrations of redox molecules according to the specific needs of the user or the treatment to be performed, provides a unique versatility to this device. Additionally, by not requiring mechanical movement of water with sodium chloride, the complexity and potential risks associated with other methods of obtaining redox molecules are reduced.

Specifically, what the invention proposes, as noted above, is a portable device for redox therapy, which comprises a container with two electrodes, preferably made of platinum, to carry out a process of electrolysis at a low dose of direct current. However, unlike other similar devices, it does not force any movement onto the water. Instead, it incorporates a type C ultraviolet light, which acts on the oxygen present between the two electrodes.

The effect of ultraviolet C light on water with sodium chloride is fundamental, as it increases the energy of oxygen, making it more reactive. This reactivity activates the formation of ozone, which becomes more abundant in the process. As a result, there is an acceleration in the creation of redox molecules, which contributes to enhancing the therapeutic benefits of the device.

Furthermore, the stability of the process is notably effective, ensuring consistent and reliable results in each redox therapy session. This innovative approach represents a significant advancement in the field of redox therapy, providing a portable and effective alternative for those seeking to enhance their wellbeing through this technology.

The process of creating redox molecules is highly scalable, as the concentration of sodium chloride used determines the different concentrations of hypochlorous acid, hydrogen peroxide, ozone, among other compounds. Depending on the intended use for these molecules, it is possible to calibrate the concentration during the extraction process.

It is important to emphasise that in this process the water must remain static, as any movement of the water would interfere with the procedure.

When electrolysis is performed at low voltage and intensity, accompanied by type C UV light in a closed container with still water, the hydrogen protons and OH groups present in the water react first. Simultaneously, sodium chloride ionises and separates into chlorine and sodium. These reactions occur while the covalent bonds of the water molecules offer resistance. After approximately an hour of maintaining the water in this condition, a stable set of redox molecules is obtained, which remain in the water for a long period. The water molecules, with a low presence of protons and hydroxyl groups, restructure, allowing the process to remain stable without immediately reversing after its completion.

The purpose of this device is to simplify the creation of redox molecules compared to the current patented systems, and to achieve a manageable model that allows this process to be carried out in a small and portable container.

The portable device for redox therapy is equipped with a programmable logic controller that plays a crucial role in controlling the operation of the electrodes and the UV radiation emitter. This controller allows precise and efficient management of the different operational parameters, ensuring a customised operation adapted to the specific needs of the users. Additionally, the device is equipped with control means that make it easier for users to adjust the operating parameters according to their individual preferences and requirements.

The programmable logic controller has the capability to be remotely linked to a control device, which can preferably be an electronic device such as a Smartphone, digital tablet, Smartwatch, or similar. On this device, a specific software application is installed that allows for communication with and remote control of the programmable logic controller. This functionality enables the management and monitoring of the controller from any location, facilitating the supervision and handling of processes controlled by the programmable logic controller. Additionally, this remote connection offers flexibility and convenience in the management of controlled devices, allowing increased efficiency in the operation and maintenance of systems where the programmable logic controller is implemented.

The use of this portable device for redox therapy offers numerous benefits. Firstly, its compact and portable design allows it to be used in different environments, making it especially useful for application in the home, medical offices, rehabilitation centres, among others. Additionally, its ease of use makes it accessible to a wide range of users, from healthcare professionals to patients wishing to benefit from the therapeutic effects of redox therapy.

Similarly, the portable device for redox therapy allows for the purification of water in an efficient and effective manner, eliminating toxins and contaminants from the water, thus improving its quality and safety for human consumption. This device makes the most of this technology, providing a practical and convenient solution for water purification. Thanks to its portable design, this device is ideal for use in different environments, from the home to commercial or industrial settings.

Unless otherwise specified, all technical and scientific terms used in this document carry their customary meaning as understood by a person skilled in the relevant field of this invention. When designing this invention, processes and materials similar or equivalent to those described may be used.

### BRIEF DESCRIPTION OF THE DRAWINGS

For the sake of completeness and to enhance comprehension of the invention's features within this description, integral and accompanying figures have been included. The figures are for illustrative purposes and are not limiting. They represent the following:

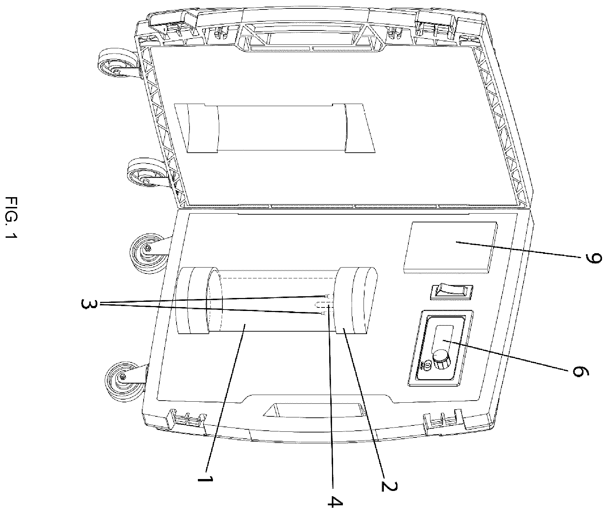

Figure 1 is a perspective representation of the portable device for redox therapy, in a briefcase-type embodiment.

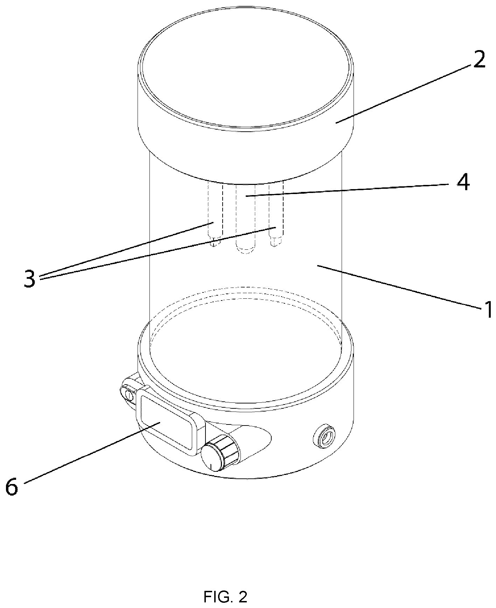

Figure 2 is a perspective representation of the portable device for redox therapy, in a tank-type embodiment.

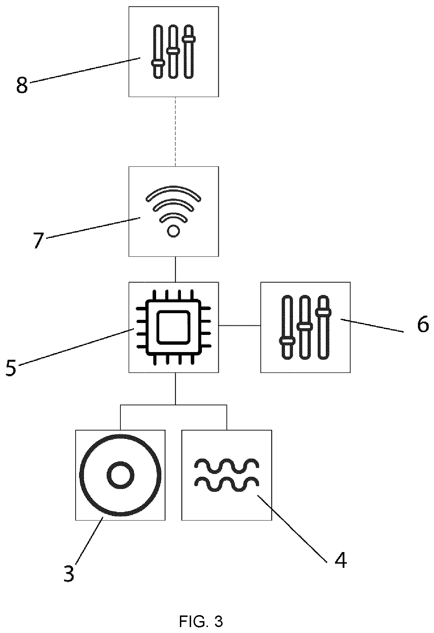

Figure 3 is a schematic representation of the electronic components of the portable device for redox therapy.

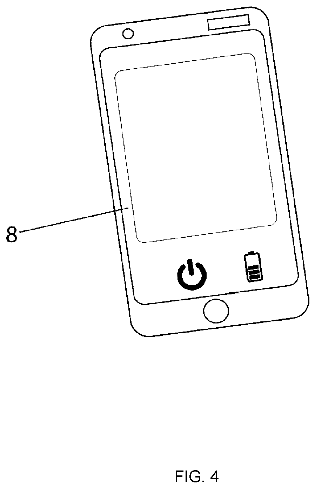

Figure 4 is a representation of the control unit (8) of the portable device for redox therapy, in a smartphone-type embodiment.

### PREFERRED USE OF THE INVENTION

In view of the figures, a preferred, though not limiting, embodiment of the proposed invention is described below, which consists of a portable device for redox therapy.

As can be seen in the figures, the portable device for redox therapy comprises a container (1) specially designed for the introduction of water with the desired amount of sodium chloride, or pre-standardised saline solution. The container (1), which ensures safety and accuracy in the process, is hermetically sealed with a lid (2) that contains the electrodes (3) necessary to carry out the redox therapy process. Additionally, the lid (2) also includes UV light emitting means (4), which play a key role in the treatment.

For a more efficient electrolysis process, the electrodes (3) are made of pure platinum, and the radiation means (4) of UV light preferentially produce type C UV radiation.

To achieve a more efficient and high-quality electrolysis process, the electrodes (3) are made of pure platinum, a material renowned for its high conductivity and resistance to corrosion. These electrodes (3) made of platinum ensure greater efficiency in charge transfer during the electrolysis process, resulting in increased productivity and reduced energy consumption. Furthermore, to optimise the performance of the electrolysis process, the UV light radiation means are preferably of type C.

On the other hand, the portable device for redox therapy comprises a programmable logic controller (5) responsible for regulating and supervising all the functions of the device, controlling the operation of the electrodes (3) and the UV light radiation means (4), in order to provide high-quality redox therapy treatments. Additionally, the programmable logic controller is connected to control means (6) from where the user can program the operation of the device.

Depending on the implementation mode, the programmable logic controller (5) may comprise communication means (7) that are linked to a control unit (8), allowing the user to programme the operation of the device from a remote location. This functionality allows the user to adjust and modify the operation of the programmable logic controller (5) as needed, without the need to be physically present at the location where the device is situated.

Additionally, the portable device for redox therapy includes at least one battery (9) as a power source. Depending on its use, the battery may be rechargeable, and the device may be connected directly to the mains for operation and/or battery recharging (9).

Additionally, the container (1) comprises an opaque coating that functions to prevent the UV radiation emitted by the radiation means from being released outside, acting as an effective barrier, ensuring that the UV radiation does not impact the surrounding environment.

Likewise, depending on the implementation mode, the control unit (8) is preferably an electronic device such as a smartphone, digital tablet, smartwatch, or similar, on which a specific software application has been installed.

The portable device for redox therapy, according to the embodiment, can take the form of a briefcase or a tank. This versatility in its configuration allows it to adapt to various needs and usage environments. Whether presented as a briefcase or as a tank, the device maintains its functionality and effectiveness to optimally provide redox therapy. This flexibility in its design makes it suitable for application in different contexts, whether for personal, clinical, or therapeutic use. Furthermore, this adaptability in its configuration allows for greater accessibility and ease of transport, which extends its reach and utility in the field of redox therapy. Regardless of whether it is used as a briefcase or as a tank, the portable device for redox therapy offers a practical and efficient solution for those looking to benefit from the positive effects of redox therapy anytime and anywhere.

Having sufficiently described the nature of this invention, as well as how to use it, further explanation is not considered necessary for any expert on the subject to understand its scope and benefits. It is also noted that, within its essential nature, the device may be implemented in other ways which differ from the one described by way of example. The protection sought shall equally apply in these cases, provided that its fundamental principal is not altered, changed or modified.

## Claims

1. Portable device for redox therapy, **characterised by** comprising at least one container (1) for the introduction of water with the desired amount of sodium chloride, or saline solution, which is hermetically sealed with a lid (2) containing electrodes (3) and UV light radiation means (4), comprising a programmable logic controller (5) that controls the operation of the electrodes (3) and UV light radiation means (4), which is connected to control means (6) from where the user can programme the operation of the device.

2. Portable device for redox therapy, according to the previous claim, **characterised in that** the electrodes (3) are made of platinum.

3. Portable device for redox therapy, according to any of the preceding claims, **characterised in that** the UV light radiation means (4) produce type C UV radiation.

4. Portable device for redox therapy, according to any of the preceding claims, **characterised in that** the programmable logic controller (5) comprises communication means (7) linked to a remote-control unit (8).

5. Portable device for redox therapy, according to the previous claim, **characterised in that** the control unit (8) is an electronic device such as a Smartphone, digital tablet, Smartwatch or similar, to which a specific software application has been installed.

6. Portable device for redox therapy, according to any of the preceding claims, **characterised in that** it comprises at least one battery (9) as a power source or is directly connected to the mains for its operation.

7. A portable device for redox therapy, according to the previous claim, **characterised in that** the battery (9) is rechargeable and is connected to the mains for recharging.

8. Portable device for redox therapy, according to any of the preceding claims, **characterised in that** the container (1) comprises an opaque coating configured to prevent UV radiation from being released to the outside.
